# EUROPÄISCHE PATENTSCHRIFT

(11) **EP 1 414 512 B1**
(45) Veröffentlichungstag und Bekanntmachung des Hinweises auf die Patenterteilung: **05.12.2007**
(21) Anmeldenummer: 02774496.0
(22) Anmeldetag: 02.08.2002
(51) Int. Cl.: A61M 25/02, A61J 15/00

(54) **MONTAGETEIL FÜR EINEN ADAPTER EINER PEG-SONDE UND ADAPTER FÜR EINE PEG-SONDE MIT EINEM DERARTIGEN MONTAGETEIL**
ASSEMBLY PART FOR AN ADAPTER OF A PEG TUBE AND ADAPTER FOR A PEG TUBE COMPRISING AN ASSEMBLY PART OF THIS TYPE
PIECE DE MONTAGE POUR UN ADAPTATEUR D'UNE SONDE DE GASTROSTOMIE ENDOSCOPIQUE PERCUTANEE ET ADAPTATEUR POUR SONDE DE GASTROSTOMIE ENDOSCOPIQUE PERCUTANEE COMPRENANT UNE TELLE PIECE DE MONTAGE

(30) Priorität: 11.08.2001 DE 10139644
(43) Veröffentlichungstag der Anmeldung: 06.05.2004
(73) Patentinhaber: Fresenius Kabi Deutschland GmbH, 61352 Bad Homburg v.d.H. (DE)
(72) Erfinder: ITRICH, Martin, 60318 Frankfurt am Main (DE); BREUER-THAL, Barbara, 65795 Hattersheim (DE)
(74) Vertreter: Luderschmidt, Schüler & Partner
(86) Internationale Anmeldenummer: PCT/EP2002/008609
(87) Internationale Veröffentlichungsnummer: WO 2003/013644

(56) Entgegenhaltungen:
- US-A- 4 473 369
- US-A- 5 549 657
- US-A- 5 792 112
- US-A1- 2001 007 067
- US-B1- 6 231 547

## Beschreibung

Die Erfindung betrifft ein Montageteil für einen Adapter zur nachträglichen Verkürzung einer PEG-Sonde, die zur künstlichen Ernährung bereits gelegt worden ist. Darüber hinaus bezieht sich die Erfindung auf einen Adapter einer PEG-Sonde mit einem derartigen Montageteil.

Zur enteralen Ernährung sind PEG-Sonden bekannt, mit denen eine Nährlösung in den Magen oder Darm des Patienten geleitet werden kann. Zum Legen einer PEG-Sonde wird ein Gastroskop oder Endoskop in den Magen des Patienten eingeführt, und der Magen wird durch Luftinsuflation entfaltet. Anschließend wird ein Kanüle durch die Bauchdecke und die Magenwand in den Magen vorgeschoben. Durch die Kanüle wird nach der klassischen Fadendurchzugsmethode ein Führungsfaden in den Magen eingeführt, mit einem Greifwerkzeug o. ä. des Gastroskops oder Endoskops erfaßt und durch Speiseröhre und Mund des Patienten wieder herausgezogen. Mit Hilfe des auf diese Weise gelegten Führungsfadens wird die Sonde dann bis zum Mageninneren und von dort durch die Punktionsstelle nach außen geführt. Dieser Eingriff wird auch als perkutane endoskopisch kontrolliere Gastrostomie (PEG) bezeichnet.

Die bekannten PEG-Sonden weisen an ihrem distalen Ende ein inneres Rückhalteglied auf, mit dem sich der Sondenschlauch an der Mageninnenwandung abstützt. Der Sondenschlauch ist derart bemessen, daß er sich weit über die Bauchdecke hinaus erstreckt. An seinem proximalen Ende weist der Schlauch einen Anschlußteil auf, um das Überleitsystem zum Zuführen von Nährlösung konnektieren zu können.

In der Praxis haben sich die bekannten PEG-Sonden bewährt. Als nachteilig wird von aktiven und mobilen Patienten aber der aus der Bauchdecke relativ weit vorstehende Sondenschlauch empfunden.

Zur Montage des Adapters wird der Sondenschlauch beispielsweise mit einer chirugischen Zange oder Klemme oberhalb der Bauchdecke fixiert und auf die erforderliche Länge gekürzt. Anschließend wird der Adapter mit dem aus der Bauchdecke vorstehenden Sondenschlauch verbunden, wobei die Fixierung mit der Zange gelöst wird. Zur Befestigung des Sondenschlauchs ist an dem Adapter ein Klemmteil vorgesehen.

Die Montage von Adaptern für PEG-Sonden der oben beschriebenen Art ist insofern erschwert, als die Klemme relativ groß und unhandlich ist, so daß die Montage behindert wird.

Die US-A-5,549,657 beschreibt einen Adapter, der eine Verkürzung des Sondenschlauchs einer bereits gelegten PEG-Sonde erlaubt, ohne die Notwendigkeit, die Sonde bei noch intaktem Sondenschlauch zu tauschen.

Der bekannte Adapter verfügt über einen Montageteil zum Fixieren der PEG-Sonde gegen Zurückrutschen. Der Montageteil weist eine zentrale Bohrung zum Durchführen der PEG-Sonde auf, in der die PEG-Sonde verklemmt wird. Der Montageteil besteht aus zwei auseinanderklappbaren Hälften, die gelenkig miteinander verbunden sind. Wenn beide Hälften des Montageteils zusammengeklappt sind, ist die Sonde gegen Zurückrutschen fixiert.

Der Erfindung liegt die Aufgabe zugrunde, einen sicher zu handhabenden Montageteil zu schaffen, der die Verkürzung einer bereits gelegten PEG-Sonde auf die erforderliche Länge und die Befestigung des Adapters an der Sonde vereinfacht. Eine weitere Aufgabe der Erfindung ist, einen Adapter mit einem derartigen Montageteil bereitzustellen.

Die Lösung dieser Aufgabe erfolgt erfindungsgemäß mit den Merkmalen der Patentansprüche 1 bzw. 9. Vorteilhafte Ausführungsformen der Erfindung sind Gegenstand der Unteransprüche.

Wenn im folgenden von einer PEG-Sonde die Rede ist, wird damit sowohl die Sonde als solche als auch der eigentliche Sondenschlauch bezeichnet.

Der erfindungsgemäße Montageteil ist als loses Distanzstück für den äußeren Rückhalteteil des Adapters ausgebildet und dient zur Fixierung der PEG-Sonde an der Bauchdecke des Patienten gegen Zurückrutschen. Vor der Montage des Adapters wird die PEG-Sonde an dem Montageteil fixiert. Damit ist die PEG-Sonde gegen Zurückrutschen gesichert. Daraufhin wird die PEG-Sonde verkürzt, und der äußere Rückhalteteils des Adapters wird an der Sonde befestigt. Vorzugsweise wird der Monategeteil auf die PEG-Sonde aufgeschoben, bis dieser auf der Bauchdecke aufliegt.

Der Montageteil kann allein oder zusammen mit dem äußeren Rückhalteteil des Adapters auf die Bauchdecke aufgeschoben werden. Vorzugsweise bildet der Montageteil mit dem äußeren Rückhalteteil eine Einheit, wobei der Montageteil nach dem Anbringen des Adapters aber von dem Rückhalteteil lösbar ist. Dadurch wird die Handhabung vereinfacht.

Der Montageteil besteht aus zwei Teilen, nämlich einem die PEG-Sonde umschließenden Haltegehäuse, das auf die Bauchdecke aufgelegt wird, und einer Halteklammer, die zur Fixierung der Sonde in dem bzw. an dem Haltegehäuse dient. Das Haltegehäuse weist vorzugsweise einen Halteteil mit einer zentralen Bohrung zum Durchführen der PEG-Sonde und einen Griffteil auf, der vorteilhafterweise mit Griffmulden versehen ist.

Die Halteklammer zur Fixierung der Sonde in dem Haltegehäuse weist einen Schlitz auf, in den die PEG-Sonde seitlich eingeschoben wird. Der Schlitz weist einen ersten Abschnitt mit einem größeren Durchmesser als die Sonde, und einen zweiten Abschnitt auf, der einen kleineren Durchmesser als die Sonde hat. Wenn sich die Sonde in dem ersten Abschnitt des Schlitzes befindet, kann der Montageteil auf der Sonde leicht verschoben werden. In dem zweiten Abschnitt des Schlitzes ist die Sonde hingegen fixiert. Damit kann die Sonde in dem Haltegehäuse allein durch Verschieben der Halteklammer festgelegt bzw. gelöst werden. Halteklammer und Haltegehäuse sind so ausgebildet, daß die Halteklammer entweder in das Haltegehäuse eingeschoben oder auf das Haltegehäuse aufgeschoben werden kann. Hierzu ist vorzugsweise eine Führung an dem Haltegehäuse vorgesehen.

Halteteil und Griffteil des Haltegehäuses bestehen vorzugsweise aus zwei Hälften, die mit einem Scharnier, insbesondere Filmscharnier miteinander verbunden sind. Dies hat den Vorteil, daß der Montageteil nach der Befestigung des Adapters leicht von der Sonde abgenommen werden kann. Die zweiteilige Ausführung des Adapters kann aber auch bei der Montage insofern vorteilhaft sein, als die Sonde nicht durch die Bohrung in dem Halteteil hindurch geführt werden braucht, sondern die Sonde einfach seitlich in das Haltegehäuse eingelegt und das Haltegehäuse durch Zusammenklappen der beiden Hälften geschlossen werden kann.

Das Haltegehäuse kann auch aus zwei nicht miteinander verbundenen Hälften bestehen, die mit der Halteklammer zusammengehalten werden. Auch kann das Haltegehäuse einteilig und mit einer Sollbruchstelle zur Teilung bei der Demontage versehen sein.

Bei einer bevorzugten Ausführungsform sind zwei Rastpositionen vorgesehen, wobei in der ersten Rastposition der erste Abschnitt des Schlitzes der Halteklammer mit der Bohrung des Haltegehäuses und in der zweiten Rastposition der zweite Abschnitt des Schlitzes der Halteklammer mit der Bohrung des Haltegehäuses fluchtet. Dies hat den Vorteil, daß die Halteklammer zwischen zwei definierten Stellungen verschoben werden kann, in denen entweder der Montageteil auf der PEG-Sonde verschiebbar oder die Sonde an dem Montageteil fixiert ist.

Nachfolgend wird ein Ausführungsbeispiel der Erfindung unter Bezugnahme auf die Zeichnungen näher erläutert.

Es zeigen:
- Fig. 1: eine Seitenansicht einer PEG-Sonde mit Adapter und Montageteil in teilweise geschnittener Darstellung,
- Fig. 2: das aufgeklappte Haltegehäuse des Montageteils der PEG-Sonde von Figur 1 in der Draufsicht,
- Fig. 3: die Halteklammer des Montageteils der PEG-Sonde von Figur 1 in der Draufsicht,
- Fig. 4: den Montageteil der PEG-Sonde von Figur 1 in der Draufsicht, wobei die Halteklammer sich in der Position befindet, in der der Montageteil auf der PEG-Sonde frei verschiebbar ist,
- Fig. 5: den Montageteil der PEG-Sonde von Figur 1 in der Draufsicht, wobei sich die Halteklammer in der die Sonde fixierenden Position befindet, und

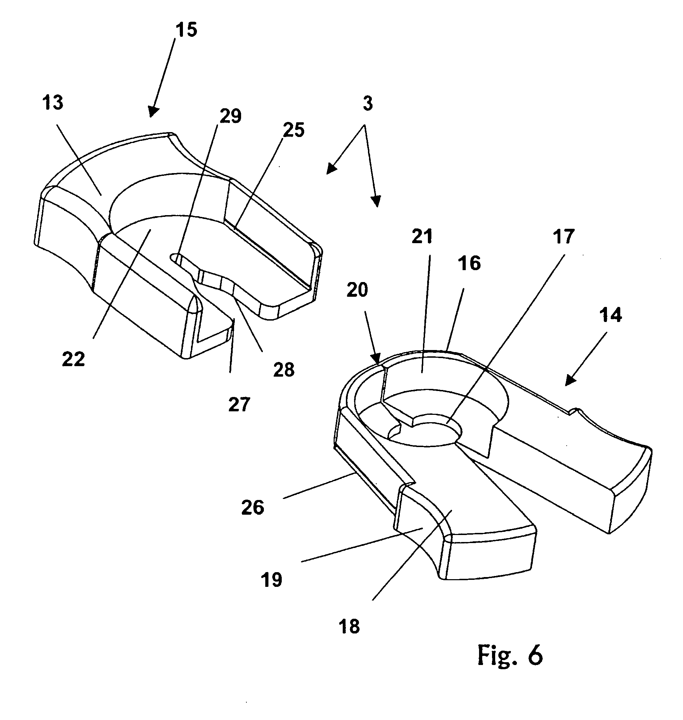

- Fig. 6: eine perspektivische Darstellung des Montageteils.

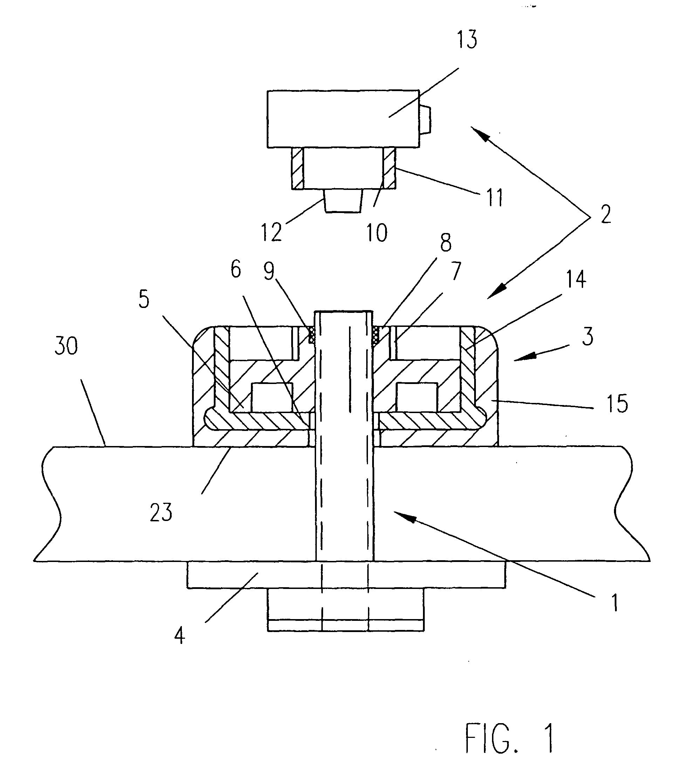

Die Figur 1 zeigt eine Seitenansicht einer PEG-Sonde 1 in teilweise geschnittener Darstellung zusammen mit einem Adapter 2, der über ein Montageteil 3 verfügt. Die PEG-Sonde 1 weist an ihrem distalen Ende einen plattenförmigen inneren Rückhalteteil 4 auf, mit dem sich die Sonde an der Mageninnenwandung abstützt. Der äußere Rückhalteteil 5, mit dem sich die Sonde an der Bauchdecke 30 abstützt, ist vorzugsweise Bestandteil des Adapters 2.

Der äußere Rückhalteteil 5 des Adapters ist eine kreisförmige Scheibe aus einem anschmiegsamen, biokompatiblen Material mit einer zentralen Öffnung 6 zum Durchführen der Sonde 1. An der dem Patienten abgewandten Oberseite weist der Rückhalteteil 5 einen mit einem Außengewinde 7 versehenen unteren hülsenförmigen Klemmteil 8 auf, in den ein elastischer Klemmring 9 eingesetzt ist, durch den die Sonde geführt wird. In einem mit einem entsprechenden Innengewinde 10 versehenen oberen hülsenförmigen Klemmteil 11 ist ein konischer Hohlzapfen 12 konzentrisch angeordnet, auf den die Sonde aufgeschoben wird.

Zum Verklemmen der Sonde 1 werden der untere und obere Klemmteil 8, 11 miteinander verschraubt, wodurch der elastische Klemmring 9 eine radiale Klemmkraft auf die Sonde ausübt.

Der obere Klemmteil 11 geht in einen nicht näher dargestellten, positiven Luer-Lock-Anschlußteil 13 oder auch einen anderen Konnektor über, an den der negative Luer-Lock-Anschlußteil des nicht dargestellten Überleitsystem zum Zuführen von Nährlösung angeschlossen werden kann. Darüber hinaus kann der Adapter 2 der Sonde beispielsweise noch über ein in die Gehäusekörper der Klemmteile integriertes Absperrorgan oder eine Verschlußkappe verfügen.

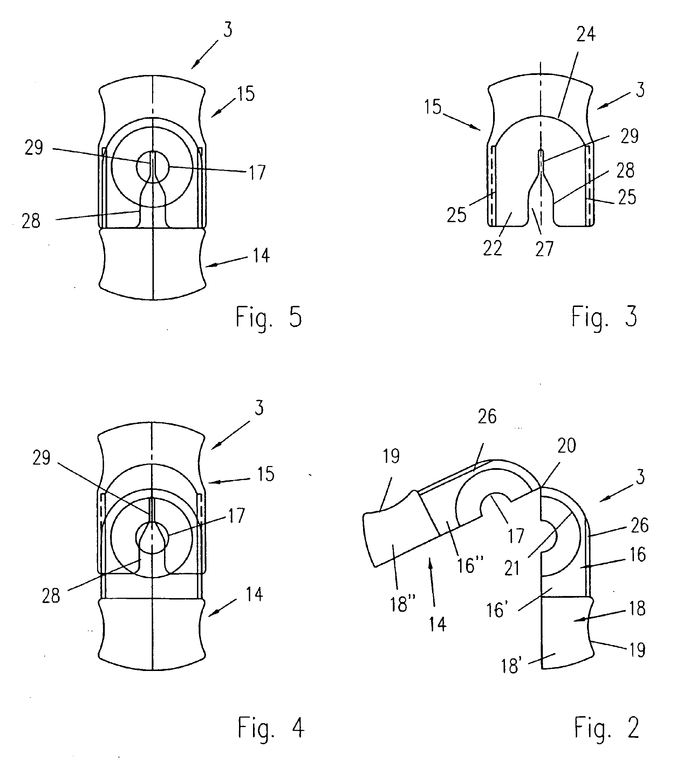

Nachfolgend wird der Montageteil 3 des Adapters 2 der PEG-Sonde 1 unter Bezugnahme auf die Figuren 2 bis 5 beschrieben. Der Montageteil 3 besteht aus einem Haltegehäuse 14 und einer Halteklammer 15.

Figur 2 zeigt das aufgeklappte Haltegehäuse 14 in der Draufsicht. Das Haltegehäuse weist einen Halteteil 16 mit einer zentralen Bohrung 17 auf, an den sich ein Griffteil 18 mit seitlichen Griffmulden 19 anschließt. Halteteil 16 und Griffteil 18 bestehen jeweils aus zwei Hälften 16', 16" und 18', 18", die mit einem Filmscharnier 20 gelenkig miteinander verbunden sind, so daß das Haltegehäuse nach der Befestigung des Adapters an der Sonde leicht demontiert werden kann.

In dem Halteteil 16 des Haltegehäuses 14 ist eine zentrale kreisförmig Ausnehmung 21 vorgesehen, deren Durchmesser so bemessen ist, daß der äußere Rückhalteteil 5 des Adapters 2 in die Ausnehmung eingesetzt und darin klemmend fixiert werden kann (Figur 1).

Figur 3 zeigt die Halteklammer 15 in der Draufsicht. Die Halteklammer 5 weist eine im wesentlichen rechteckförmige Grundplatte 22 mit abgerundeten Ecken auf, die mit ihrer flachen Unterseite 23 auf die Bauchdecke 30 des Patienten aufgelegt werden kann (Figur 1). Entlang der beiden Längsseiten und einer der Stirnseiten der Grundplatte 22 verläuft ein umlaufender Rand 24. Die Abmessungen des Haltegehäuses 14 und der Halteklammer 15 sind derart bemessen, daß der Halteteil 16 des Haltegehäuses 14 passend in die Halteklammer 15 eingeschoben werden kann. Dabei bilden längslaufende Nuten 25 in dem umlaufenden Rand 24 der Halteklammer 15 und längslaufende Nasen 26 an dem Halteteil 16 des Haltegehäuses 14 eine Führung. Die Figuren 4 und 5 zeigen die zusammengeschobenen Teile des Montageteils 3 in der Draufsicht.

In der Grundplatte 22 der Halteklammer 15 erstreckt sich ein längslaufender Schlitz 27, dessen Durchmesser sich in Längsrichtung verringert. Der Schlitz 27 weist einen ersten Abschnitt 28 mit einem größeren Durchmesser als die Sonde 1 und einen zweiten Abschnitt 29 mit einem kleineren Durchmesser als die Sonde 1 auf.

Die Führung von Haltegehäuse 14 und Halteklammer 15 weist zwei Rastpositionen auf. Die Halteklammer kann in dem Haltegehäuse beispielsweise durch Nasen und entsprechende Mulden einrastend fixiert werden. In der ersten Rastposition ist die Halteklammer nur soweit in das Haltegehäuse eingeschoben, daß der erste Abschnitt 28 des Schlitzes 27 in der Halteklammer 15, der einen größeren Durchmesser als die Sonde hat, mit der zentralen Bohrung 17 in dem Haltegehäuse 14 fluchtet, so daß der Montageteil 3 auf der Sonde frei verschiebbar ist (Figur 4). In der zweiten Rastposition fluchtet der zweite Abschnitt 29 des Schlitzes 27, der einen kleineren Durchmesser als die Sonde hat, mit der zentralen Bohrung 17, so daß die Sonde in dem Montageteil fixiert ist (Fig. 5).

Die Montage des Adapters 2 der PEG-Sonde 1 kann wie folgt vorgenommen werden. Zur Montage des Adapters wird zunächst die Sonde 1 in die Bohrung 17 des Haltegehäuses 14 eingelegt, und das Haltegehäuse wird zusammengeklappt. Anschließend wird die Halteklammer 15 bis zu der ersten Rastposition auf das Haltegehäuse aufgeschoben (Fig. 4). Der Montageteil 3 des Adapters wird nun soweit auf der Sonde vorgeschoben, bis die Unterseite 23 des Halteklammer 15 flach auf der Bauchdecke 30 aufliegt. Daraufhin wird die Halteklammer auf dem Haltegehäuse in die zweite Rastposition vorgeschoben, so daß die Sonde gegen Zurückrutschen gesichert ist (Fig. 5). Das überstehende Ende der Sonde wird jetzt unmittelbar oberhalb des Montageteils 3 abgeschnitten.

Vorzugsweise wird der Adapter aber wie folgt montiert. Bei der bevorzugten Ausführungsform sitzt der äußere Rückhalteteil 5 des Adapters 2 vor der Montage bereits leicht klemmend in der Ausnehmung 21 des in die Halteklammer 15 bereits eingesetzten Haltegehäuses 14, wobei sich die Halteklammer in der ersten Rastposition befindet. Die Sonde wird in die Bohrung 17 eingeführt und der Montageteil wird zusammen mit der Rückhalteplatte des Adapters auf die Bauchdecke vorgeschoben. Anschließend wird die Sonde mit dem Montageteil fixiert und auf die erforderliche Länge abgeschnitten..

Der zylindrische Konus 12 des zweiten Klemmteils 11 des Adapters 2 wird daraufhin in die Sonde 1 eingeführt, und das Haltegehäuse 14 bzw. die Halteklammer 15 wird anschließend in die erste Rastposition zurückgeschoben, so daß zwar die beiden Hälften des Haltegehäuses noch zusammengehalten, die Sonde aber schon frei beweglich ist. Im Anschluß daran wird der obere Klemmteil 11 mit dem unteren Klemmteil 8 des Adapters 2 verschraubt, wobei der untere Klemmteil 8 mit dem äußeren Rückhalteteil 5 festgehalten und der obere Klemmteil 10 gedreht wird.

Nunmehr wird die Halteklammer von dem Haltegehäuse abgezogen und das Haltegehäuse wird nach dem Aufklappen der beiden Gehäusehälften entfernt. Da die Montageplatte als loses Distanzstück dient, das einen Abstand zwischen dem äußeren Rückhalteteil 5 des Adapters 2 und der Bauchdecke 30 von etwa 5 bis 7 mm schafft, hat die Sonde 1 ausreichendes Spiel.

Alternativ kann auch der äußere Rückhalteteil 5 des Adapters 2 mit dem unteren Klemmteil 8 gedreht und der obere Klemmteil 11 des Adapters festgehalten werden. Dadurch wird ein Drehen der Sonde im Stomakanal vermieden.

Der erfindungsgemäße Montageteil erlaubt eine einfache und sichere Montage des PEG-Adapters. Fehler durch Abschneiden der Sonde an der falschen Stelle, was zu einer falschen Sondenlänge führt, können vermieden werden. Der Montageteil erleichtert das sichere Halten des äußeren Rückhalteteils und des unteren Klemmteils des Adapters und vermeidet so eine Druckbelastung der Bauchdecke des Patienten. Wenn der Montageteil mit dem Adapter eine lösbare Einheit bildet, wird die Handhabung noch weiter vereinfacht.

## Patentansprüche

1. Montageteil für einen Adapter einer PEG-Sonde, die einen sich an der Magenwandung abstützenden inneren Rückhalteteil an ihrem distalen Ende und ein offenes proximales Ende aufweist, wobei der Adapter einen sich an der Bauchdecke des Patienten abstützenden äußeren Rückhalteteil mit Mitteln zum Anschluß einer PEG-Sonde und Mitteln zum Anschluß eines Überleitsystems an die Sonde aufweist, und wobei der Montageteil (3) zum Fixieren der PEG-Sonde gegen Zurückrutschen ein die PEG-Sonde umschließendes Haltegehäuse (14) mit einer zentralen Bohrung (17) zum Durchführen der PEG-Sonde aufweist,
**dadurch gekennzeichnet,**
**daß** der Montageteil (3) eine Halteklammer (15) mit einem Schlitz (27) zum seitlichen Einschieben der PEG-Sonde aufweist, wobei der Schlitz einen ersten Abschnitt (28) mit einem größeren Durchmesser als die Sonde aufweist, in dem die Sonde frei beweglich ist, und einen zweiten Abschnitt (29) mit einem kleineren Durchmesser als die Sonde aufweist, in dem die Sonde fixiert ist, und
**daß** das Haltegehäuse (14) eine Führung (25, 26) zum Einschieben oder Aufschieben der Halteklammer (15) in das bzw. auf das Haltegehäuse aufweist.

2. Montageteil nach Anspruch 1, **dadurch gekennzeichnet, daß** das Haltegehäuse (14) einen Halteteil (16) mit der zentralen Bohrung (17) zum Durchführen der PEG-Sonde und einen Griffteil (18) aufweist.

3. Montageteil nach Anspruch 2, **dadurch gekennzeichnet, daß** der Griffteil (18) des Haltegehäuses (14) seitliche Griffmulden (19) aufweist.

4. Montageteil nach einem der Ansprüche 1 bis 3, **dadurch gekennzeichnet, daß** der Halteteil (16) und der Griffteil (18) des Haltegehäuses (14) aus zwei auseinanderklappbaren Hälften (16', 16", 18', 18") bestehen.

5. Montageteil nach Anspruch 4, **dadurch gekennzeichnet, daß** die Hälften (16', 16", 18', 18") des Halteteils (16) und Griffteils (18) mit einem Scharnier (20), insbesondere Filmscharnier miteinander gelenkig verbunden sind.

6. Montageteil nach einem der Ansprüche 1 bis 5, **dadurch gekennzeichnet, daß** das Haltegehäuse (14) eine Ausnehmung (21) zum Einsetzen des äußeren Rückhalteteils des Adapters aufweist.

7. Montageteil nach Anspruch 6, **dadurch gekennzeichnet, daß** die Ausnehmung (21) zur klemmenden Fixierung des äußeren Rückhalteteils ausgebildet ist.

8. Montageteil nach einem der Ansprüche 1 bis 7, **dadurch gekennzeichnet, daß** die Führung (25, 26) des Haltegehäuses (14) eine erste und zweite Rastposition aufweist, wobei in der ersten Rastposition der erste Abschnitt (28) des Schlitzes (27) der Halteklammer (15) mit der Bohrung (17) des Haltegehäuses (14) und in der zweiten Rastposition der zweite Abschnitt (29) des Schlitzes der Halteklammer mit der Bohrung des Haltegehäuses fluchtet.

9. Adapter für eine PEG-Sonde mit einem sich an der Magenwandung abstützenden inneren Rückhalteteil an ihrem distalen Ende und einem offenen proximalen Ende, wobei der Adapter einen sich an der Bauchdecke des Patienten abstützenden äußeren Rückhalteteil mit Mitteln zum Anschluß der PEG-Sonde und Mitteln zum Anschluß eines Überleitsystems an die Sonde aufweist, **dadurch gekennzeichnet, daß** der Adapter ein Montageteil nach einem der Ansprüche 1 bis 8 aufweist.

## Claims

1. Fitting-facilitating part for an adapter for a PEG probe, which probe has an inner retaining member located at its distal end and supported against the wall of the stomach and an open proximal end, the adapter having an outer retaining part which is supported on the patient's abdominal wall and which has means for connection to a PEG probe and means for connecting a delivery system to the probe, and the fitting-facilitating part (3) having, to secure the PEG probe against slipping back, a holding housing (14) which surrounds the PEG probe and has a central bore (17) for the PEG probe to pass through, **characterised in that** the fitting-facilitating part (3) has a holding clip (15) having a slot (27) for sideways insertion of the PEG probe by sliding, the slot having a first portion (28) of larger diameter than the probe in which the probe is freely movable and a second portion (29) of smaller diameter than the probe in which the probe is secured, and **in that** the holding housing (14) has guiding means (25, 26) to allow the holding clip (15) to be slid into or onto the holding housing.

2. Fitting-facilitating part according to claim 1, **characterised in that** the holding housing (14) has a holding part (16) having the central bore (17) for the PEG probe to pass through, and a handle part (18).

3. Fitting-facilitating part according to claim 2, **characterised in that** the handle part (18) of the holding housing (14) has lateral depressions (19) to provide a grip.

4. Fitting-facilitating part according to one of claims 1 to 3, **characterised in that** the holding part (16) and the handle part (18) of the holding housing (14) comprise two halves (16', 16", 18', 18") able to be folded apart from one another.

5. Fitting-facilitating part according to claim 4, **characterised in that** the halves (16', 16", 18', 18") of the holding part (16) and the handle part (18) are connected together in such a way as to hinge by a hinge (20), in particular a film hinge.

6. Fitting-facilitating part according to one of claims 1 to 5, **characterised in that** the holding housing (14) has a recess (21) for the insertion of the outer retaining part of the adapter.

7. Fitting-facilitating part according to claim 6, **characterised in that** the recess (21) is designed to secure the outer retaining part in position by clamping.

8. Fitting-facilitating part according to one of claims 1 to 7, **characterised in that** the guiding means (25, 26) of the holding housing (14) have a first and a second latched position, the first portion (28) of the slot (27) in the holding clip (15) lining up with the bore (17) in the holding housing (14) in the first latched position, and the second portion (29) of the slot in the holding clip lining up with the bore in the holding housing in the second latched position.

9. Adapter for a PEG probe having an inner retaining member located at its distal end and supported against the wall of the stomach and an open proximal end, the adapter having an outer retaining part which is supported on the patient's abdominal wall and which has means for connection to a PEG probe and means for connecting a delivery system to the probe, **characterised in that** the adapter has a fitting-facilitating part according to one of claims 1 to 8.

## Revendications

1. Pièce de montage pour un adaptateur d'une sonde de gastrostomie endoscopique percutanée (GEP) qui présente une partie de retenue interne s'appuyant sur la paroi de l'estomac, au niveau de son extrémité distale, et une extrémité proximale ouverte, l'adaptateur présentant une partie de retenue externe s'appuyant sur la paroi abdominale du patient avec des moyens de raccordement d'une sonde de GEP et des moyens de raccordement d'un système de dérivation à la sonde, et la pièce de montage (3) présentant, pour la fixation de la sonde de GEP contre les glissements, un boîtier de retenue (14) entourant la sonde de GEP, doté d'un alésage central (17) pour introduction de la sonde de GEP,
**caractérisée en ce que**
la pièce de montage (3) présente un clip de maintien (15) doté d'une fente (27) pour insertion latérale de la sonde de GEP, la fente présentant un premier tronçon (28) présentant un diamètre supérieur à celui de la sonde, dans lequel la sonde peut être déplacée librement, et un deuxième tronçon (29) présentant un diamètre inférieur à celui de la sonde, dans lequel la sonde est fixée, et
**en ce que** le boîtier de retenue (14) présente un guidage (25, 26) pour insérer ou arrêter le clip de maintien (15) dans ou sur le boîtier de retenue.

2. Pièce de montage selon la revendication 1, **caractérisée en ce que** le boîtier de retenue (14) présente une partie de retenue (16) dotée de l'alésage central (17) pour introduire la sonde de GEP et une partie de préhension (18).

3. Pièce de montage selon la revendication 2, **caractérisée en ce que** la partie de préhension (18) du boîtier de retenue (14) présente des creux de préhension latéraux (19).

4. Pièce de montage selon l'une quelconque des revendications 1 à 3, **caractérisé en ce que** la partie de retenue (16) et la partie de préhension (18) du boîtier de retenue (14) sont constituées de deux moitiés pouvant être ouvertes (16', 16'', 18', 18'').

5. Pièce de montage selon la revendication 4, **caractérisée en ce que** les moitiés (16', 16'', 18', 18'') de la partie de retenue (16) et la partie de préhension (18) sont reliées de façon articulée par une charnière (20), en particulier une charnière à film.

6. Pièce de montage selon l'une quelconque des revendications 1 à 5, **caractérisée en ce que** le boîtier de retenue (14) présente un évidement (21) pour placer la partie de retenue externe de l'adaptateur.

7. Pièce de montage selon la revendication 6, **caractérisée en ce que** l'évidement (21) est constitué pour la fixation par clip de la partie de retenue externe.

8. Pièce de montage selon l'une quelconque des revendications 1 à 7, **caractérisée en ce que** le guidage (25, 26) du boîtier de retenue (14) présente une première et une deuxième position d'arrêt, dans laquelle, dans la première position d'arrêt, le premier tronçon (28) de la fente (27) du clip de maintien (15) est aligné avec l'alésage (17) du boîtier de retenue (14) et, dans la deuxième position d'arrêt, le deuxième tronçon (29) de la fente du clip de maintien du clip de maintien est aligné avec l'alésage du boîtier de retenue.

9. Adaptateur pour une sonde de GEP qui présente une partie de retenue interne s'appuyant sur la paroi de l'estomac, au niveau de son extrémité distale et une extrémité proximale ouverte, l'adaptateur présentant une partie de retenue externe s'appuyant sur la paroi abdominale du patient avec des moyens de raccordement de la sonde de GEP et des moyens de raccordement d'un système de dérivation à la sonde, **caractérisée en ce que** l'adaptateur présente une pièce de montage selon l'une quelconque des revendication 1 à 8.
